# EUROPEAN PATENT APPLICATION

(11) **EP 3 744 314 A1**
(43) Date of publication of application: **02.12.2020**
(21) Application number: 20176717.5
(22) Date of filing: 27.05.2020
(51) Int. Cl.: A61K 9/00, A61K 9/20, A61K 31/167, A61K 31/522

(54) **METHOD FOR PRODUCING TABLET**

(30) Priority: 31.05.2019 JP 2019102984
(71) Applicant: Shin-Etsu Chemical Co., Ltd., Tokyo 100-0004 (JP)
(72) Inventor: HIRAMA, Yasuyuki, NIIGATA, 942-8601 (JP)
(74) Representative: Nony

(57) **Abstract**

There is provided a method for producing a tablet having high tablet hardness without delaying the disintegration time. More specifically, there is provided a method for producing a tablet, including a granulation step of granulating a powder composition containing one or more selected from the group consisting of an active ingredient, an excipient and a disintegrant, while adding an aqueous composition containing a binder and water to the powder composition, to obtain a granulated product; and a heat treatment and tableting step of subjecting the granulated product to heat treatment and then tableting, or to tableting the granulated product and then heat treatment, to obtain a tablet.

## Description

### BACKGROUND OF THE INVENTION

### 1. Field of the Invention

The invention relates to a method for producing a tablet, which is one of the dosage forms of solid preparation in the field of pharmaceuticals or foods.

### 2. Related Art

A tablet, which is one of solid dosage forms of solid preparation in the field of pharmaceuticals or foods, is a solid preparation obtained by compression-molding powder into a predetermined shape, and has advantages such as easy handling. Especially in the field of pharmaceuticals, the tablets occupy about 50% in all products, and are most widely used.

Examples of the method for producing tablets include a direct compression method, a dry granulation tableting method, an extrusion granulation tableting method, and a wet granulation tableting method. Above all, the wet granulation tableting method is widely used both in Japan and overseas because the granulation in the method can greatly improve binding properties and flowability of powder as well as uniformity of the active ingredient content in each tablet despite the complication of the method, compared with the dry direct tableting method in which an active ingredient, an excipient and so on are mixed and tableted as they are.

The wet granulation tableting method comprises steps of: granulating a mixture of an active ingredient and an excipient, while spraying or adding a solution of a binder or an appropriate solvent such as water or ethanol to obtain a granulated product; drying the granulated product; and tableting the dried granulated product to obtain tablets. Examples of the wet granulation tableting method include a high shear wet granulation tableting method by using a high shear granulator is used; and a fluidized bed granulation tableting method using a fluidized bed granulator.

When the granulation is carried out using only a solvent such as water or ethanol without using a solution of a binder in the wet granulation tableting method, a tablet having the desired tablet hardness may not be obtained, or tableting failure such as capping, laminating or sticking may occur. Hence, the granulation is generally carried out by using a solution of a binder such as polyvinyl alcohol, hydroxypropyl cellulose, polyvinylpyrrolidone, or hydroxypropyl methyl cellulose. However, when the amount of the binder added is increased to increase the tablet hardness, there is a problem that the disintegration of the obtained tablet is deteriorated.

Therefore, it is desired to develop a method for producing a tablet having a high tablet hardness without increasing the amount of a binder and without using a special technique or equipment.

For example, JPH07-503237A provides a method of producing a tablet with increased strength, comprising steps of: (a) compressing a mixture of a meltable binder, at least one excipient and a pharmaceutically active agent into a tablet; (b) melting the binder in the tablet; and (c) solidifying the binder.

Furthermore, JP2004-292457A provides a method for producing a orally rapidly disintegrating tablet comprising a drug, a diluent, and erythritol, which is a saccharide having a relatively lower melting point than the drug and the diluent, comprising steps of: (a) compression-molding a starting material containing the drug, the diluent, the erythritol and a binder which is a saccharide having high moldability and/or a water-soluble polymer at a low pressure required for maintaining the tablet shape, (b) heating the molded product obtained in step (a) to a temperature higher than or equal to the temperature at which the erythritol melts, and (c) cooling the molded product obtained in step (b) to a temperature lower than or equal to the temperature at which the melted erythritol solidifies.

### SUMMARY OF THE INVENTION

However, in the method of JPH07-503237A, there have been cases in which the tablet hardness and disintegration time are changed when the tablet is stored in a room where the air-conditioning facility is inadequate. It is because the binder is remelted by the increased temperature in the room mainly during summer. In the method of JP2004-292457A, there have been cases in which the disintegration time is delayed despite the attempt to improve the tablet hardness by using dissolution and solidification of erythritol.

The invention has been made in view of the above circumstances, and an object of the invention is to provide a method for producing a tablet excellent in hardness and/or disintegratability without delaying disintegration time of the tablet.

As a result of intensive studies to achieve the above object, the inventors have found that a powder composition containing at least one selected from the group consisting of an active ingredient, an excipient and a disintegrant is granulated, while adding an aqueous composition containing water and a binder selected from the group consisting of polyvinyl alcohol, hydroxypropyl cellulose, polyvinylpyrrolidone, hydroxypropyl methyl cellulose, methyl cellulose, sodium carboxymethylcellulose, polyvinyl alcohol/acrylic acid/methyl methacrylate copolymers, and vinylpyrrolidone/vinyl acetate (VP/VA) copolymers thereto to obtain a granulated product; and the granulated product is heat-treated followed by tableting, or is tableted followed by heat treatment, to obtain a tablet having excellent hardness and/or disintegratabililty without delaying the disintegration time.

In an aspect of the invention, there is provided a method for producing a tablet comprising: a granulation step of granulating a powder composition comprising at least one selected from the group consisting of an active ingredient, an excipient and a disintegrant, while adding an aqueous composition comprising a binder and water to the powder composition, to obtain a granulated product; and a heat treatment and tableting step of subjecting the granulated product to heat treatment and then tableting, or to tableting and then heat treatment, to obtain a tablet; wherein the binder is selected from the group consisting of polyvinyl alcohol, hydroxypropyl cellulose, polyvinylpyrrolidone, hydroxypropyl methyl cellulose, methyl cellulose, sodium carboxymethylcellulose, polyvinyl alcohol/acrylic acid/methyl methacrylate copolymers, and vinyl pyrrolidone/vinyl acetate copolymers.

According to the invention, a high-quality tablet excellent in hardness and/or disintegration without delaying the disintegration time can be produced, so that the occurrence of cracking or chipping in the tablet can be suppressed during packing or transportation of the tablet, and the medicinal effect can be quickly developed when the tablet is orally administered.

### DETAILED DESCRIPTION OF THE PREFERRED EMBODIMENTS

The granulation step of granulating a powder composition comprising at least one selected from the group consisting of an active ingredient, an excipient and a disintegrant, while adding an aqueous composition comprising a binder and water to the powder composition, to obtain a tablet, will be described.

The powder composition contains at least one selected from the group consisting of an active ingredient, an excipient and a disintegrant. When the powder composition does not contain the active ingredient, the aqueous composition may contain the active ingredient, or a step or substep of mixing the granulated product with the active ingredient may be provided between the granulation step and the heat treatment and tableting step, or may be provided after the heat treatment and before the tableting in the heat treatment and tableting step.

The active ingredient is not particularly limited as long as it is an active ingredient that can be orally administered. Examples of the active ingredient include drugs used in pharmaceutical products and active ingredients used in health foods such as foods with nutrient function claims, foods for specified health use, and foods with functional claims.

Example of the drug used in pharmaceutical products include a drug for the central nervous system, a drug for the cardiovascular system, a drug for the respiratory system, a drug for the digestive system, an antibiotic, an antitussive and expectorant, an antihistamine, an antipyretic anti-inflammatory analgesic, a diuretic, an autonomic agent, an antimalarial agent, an antidiarrheal agent, a psychotropic, and vitamins and derivatives thereof.

Examples of the drug for the central nervous system include diazepam, idebenone, naproxen, piroxicam, indomethacin, sulindac, lorazepam, nitrazepam, phenytoin, acetaminophen, ethenzamide, ketoprofen, and chlordiazepoxide.

Examples of the drug for the cardiovascular system include molsidomine, vinpocetine, propranolol, methyldopa, dipyridamole, furosemide, triamterene, nifedipine, atenolol, spironolactone, metoprolol, pindolol, captopril, isosorbide dinitrate, delapril hydrochloride, meclofenoxate hydrochloride, diltiazem hydrochloride, etilefrine hydrochloride, digitoxin, and alprenolol hydrochloride.

Examples of the drug for the respiratory system include amlexanox, dextromethorphan, theophylline, pseudoephedrine, salbutamol, and guaifenesin.

Examples of the drug for the digestive system include a benzimidazole drug having antiulcer action, such as 2-[[3-methyl-4-(2,2,2-trifluoroethoxy)-2-pyridyl]methylsulfinyl]benzimidazole and 5-methoxy-2-[4-methoxy-3,5-dimethyl-2-pyridyl)methylsulfinyl]benzimidazole; cimetidine; ranitidine; pirenzepine hydrochloride; pancreatin; bisacodyl; and 5-aminosalicylic acid.

Examples of the antibiotic include talampicillin hydrochloride, bacampicillin hydrochloride, cefaclor, and erythromycin.

Examples of the antitussive and expectorant include noscapine hydrochloride, carbetapentane citrate, isoaminile citrate, and dimemorfan phosphate.

Examples of the antihistamine include chlorpheniramine maleate, diphenhydramine hydrochloride, and promethazine hydrochloride.

Examples of the antipyretic anti-inflammatory analgesic include ibuprofen, diclofenac sodium, flufenamic acid, sulpyrine, aspirin, and ketoprofen.

Examples of the diuretic include caffeine.

Examples of the autonomic agent include dihydrocodeine phosphate, dl-methylephedrine hydrochloride, atropine sulfate, acetylcholine chloride, and neostigmine.

Examples of the antimalarial agent include quinine hydrochloride.

Examples of the antidiarrheal agent include loperamide hydrochloride.

Examples of the psychotropic include chlorpromazine.

Examples of the vitamins and derivatives thereof include vitamin A, vitamin B1, fursultiamine, vitamin B2, vitamin B6, vitamin B12, vitamin C, vitamin D, vitamin E, vitamin K, calcium pantothenate, and tranexamic acid.

Examples of the active ingredient used in the health food include the above vitamins and derivatives thereof, minerals, carotenoids, amino acids and derivatives thereof, plant extracts, and health food materials.

Examples of the mineral include calcium, magnesium, manganese, zinc, iron, copper, selenium, chromium, sulfur, and iodine.

Examples of the carotenoid include β-carotene, α-carotene, lutein, cryptoxanthin, zeaxanthin, lycopene, astaxanthin, and multicarotene.

Examples of the amino acid include an acidic amino acid, a basic amino acid, a neutral amino acid, and an acidic amino acid amide.

Examples of the acidic amino acid include aspartic acid and glutamic acid.

Examples of the basic amino acid include lysine, arginine, and histidine.

Examples of the neutral amino acid include linear aliphatic amino acids such as alanine and glycine; branched aliphatic amino acids such as valine, leucine and isoleucine; hydroxyamino acids such as serine and threonine; sulfur-containing amino acids such as cysteine and methionine; aromatic amino acids such as phenylalanine and tyrosine; heterocyclic amino acids such as tryptophan; and imino acids such as proline.

Examples of the acidic amino acid amide include asparagine and glutamine.

Examples of the amino acid derivative include acetylglutamine, acetylcysteine, carboxymethylcysteine, acetyltyrosine, acetylhydroxyproline, 5-hydroxyproline, glutathione, creatine, S-adenosylmethionine, glycylglycine, glycylglutamine, dopa, alanylglutamine, carnitine and γ-aminobutyric acid.

Examples of the plant extract include aloe extract, propolis extract, agaricus extract, Panax ginseng extract, ginkgo leaf extract, turmeric extract, curcumin, sprouted brown rice extract, shiitake mycelium extract, Rubus suavissimus extract, sweet Hydrangea leaf extract, Fomes yucatensis extract, sesame extract, garlic extract, maca (Lepidium meyenii) extract, plant worm (Cordyceps sinensis) extract, camomile extract, and red pepper extract.

Examples of the health food material include royal jelly; dietary fiber; proteins; bifidobacteria; lactic acid bacteria; chitosan; yeast; glucosamine; lecithin; polyphenols; cartilage of animals, fish and shellfish; soft-shelled turtle; lactoferrin; freshwater clams; eicosapentaenoic acid; germanium; enzymes; creatine; carnitine; citric acid; raspberry ketone; coenzyme Q10; methylsulfonylmethane; and soybean peptides bonded with phospholipids.

An amount of the active ingredient may be determined, depending on the content of the active ingredient in the tablet described later. If necessary, two or more types of active ingredients may be used. A commercially available active ingredient may be used.

Examples of the excipient include sugars such as white soft sugar, lactose, glucose and maltose; sugar alcohols such as D-mannitol, sorbitol and maltitol; starches such as wheat starch, rice starch, potato starch and corn starch; dextrins; powdered cellulose; microcrystalline cellulose; calcium carbonate; calcium phosphate; and calcium sulfate.

An amount of the excipient may be determined, depending on the content of the active ingredient in the tablet described later. If necessary, two or more types of excipients may be used. A commercially available excipient may be used.

Examples of the disintegrant include low-substituted hydroxypropyl cellulose; starches such as wheat starch, rice starch, potato starch and corn starch; partly pregelatinized starch; sodium starch glycolate; carmellose; carmellose calcium; croscarmellose sodium; microcrystalline cellulose; and crospovidone.

The content of hydroxypropoxy groups in the low-substituted hydroxypropyl cellulose is preferably from 5 to 16% by mass, more preferably from 7 to 15% by mass, from the viewpoint of disintegration. The content of the hydroxypropoxy groups in the low-substituted hydroxypropyl cellulose may be determined by the assay described in the section "Low-substituted hydroxypropyl cellulose" of the Japanese Pharmacopoeia Seventeenth Edition.

An amount of disintegrant may be determined, depending on the content of the disintegrant in the intended tablet. If necessary, two or more types of disintegrants may be used. A commercially available disintegrant may be used.

The aqueous composition comprises a binder and water.

The binder is selected from the group consisting of polyvinyl alcohol, hydroxypropyl cellulose, polyvinylpyrrolidone, hydroxypropyl methyl cellulose, methyl cellulose, sodium carboxymethylcellulose, polyvinyl alcohol/acrylic acid/methyl methacrylate copolymers, and vinyl pyrrolidone/vinyl acetate (VP/VA) copolymers.

The above binder is a solid binder having no melting point preferably in the range of from 25 to 180°C, and the hardness and/or disintegration of the tablet is considered to be improved by developing softening or changing the orientation (crystallinity) in the crystal structure of the binder at a temperature of preferably higher than 80° C.

The degree of saponification of the polyvinyl alcohol is preferably 78.0 mol% or more, more preferably from 85.0 to 99.5 mol%, from the viewpoint of solubility in water. The degree of saponification of the polyvinyl alcohol may be determined in accordance with the method of determining the degree of saponification described in JIS K6726.

The viscosity at 20°C of a 4% by mass aqueous solution of the polyvinyl alcohol is preferably from 2.0 to 100.0 mPa·s, more preferably from 3.0 to 50.0 mPa·s, from the viewpoint of binding and disintegration. The viscosity at 20°C of a 4% by mass aqueous solution of the polyvinyl alcohol may be determined in accordance with the method of determining viscosity described in JIS K6726.

The content of hydroxypropoxy groups in the hydroxypropyl cellulose is not particularly limited. It is preferably from 53.4 to 80.5% by mass, more preferably from 55.0 to 75.0% by mass. The content of hydroxypropoxy groups in the hydroxypropyl cellulose may be determined by the quantitative method described in the section "Hydroxypropyl cellulose" of the Japanese Pharmacopoeia Seventeenth Edition.

The K-value of polyvinylpyrrolidone is not particularly limited. It is preferably from 10 to 120, more preferably from 15 to 100. The K-value of polyvinylpyrrolidone may be determined by the method for determining K-value described in the section "Povidone" of the Japanese Pharmacopoeia Seventeenth Edition.

The content of methoxy groups in the hydroxypropyl methyl cellulose is not particularly limited. It is preferably from 16.5 to 30.0% by mass, more preferably from 19.0 to 30.0% by mass. The content of hydroxypropoxy groups in the hydroxypropyl methyl cellulose is not particularly limited. It is preferably from 4.0 to 32.0% by mass, more preferably from 4.0 to 12.0% by mass. The content of methoxy groups and the content of hydroxypropoxy groups in the hydroxypropyl methyl cellulose may be determined by the assay method described in the section "Hypromellose" of the Japanese Pharmacopoeia Seventeenth Edition.

The viscosity at 20°C of a 2% by mass aqueous solution of hydroxypropyl methyl cellulose is preferably from 2.0 to 100.0 mPa·s, more preferably from 3.0 to 50.0 mPa·s, from the viewpoint of binding and disintegration. The viscosity at 20°C of a 2% by mass aqueous solution of hydroxypropyl methyl cellulose may be determined by the method for determining viscosity described in the section "Hypromellose" of the Japanese Pharmacopoeia Seventeenth Edition.

The content of the methoxy group in the methyl cellulose is not particularly limited. It is preferably from 26.0 to 33.0% by mass. The content of the methoxy group in the methylcellulose may be determined by the quantitative method described in the section "Methyl cellulose" of the Japanese Pharmacopoeia Seventeenth Edition.

The viscosity at 20°C of a 2% by mass aqueous solution of the methylcellulose is preferably from 2.0 to 100.0 mPa·s, more preferably from 3.0 to 50.0 mPa·s, from the viewpoint of binding and disintegration. The viscosity at 20°C of a 2% by mass aqueous solution of the methyl cellulose may be determined by the method for determining viscosity described in the section "Methyl cellulose" of the Japanese Pharmacopoeia Seventeenth Edition.

The content of sodium in the sodium carboxymethylcellulose is not particularly limited. It is preferably from 6.5 to 8.5% by mass. The content of sodium in the sodium carboxymethylcellulose may be determined by the quantitative method described in the section "sodium carboxymethylcellulose" of the Japanese Pharmacopoeia Seventeenth Edition.

As the binder, polyvinyl alcohol is preferable from the viewpoint of improvement of the disintegration time.

If necessary, two or more types of binders may be used. A commercially available binder may be used. An amount of the binder is described below as the content in the tablet.

Examples of the water include purified water.

An amount of the water is not particularly limited. It is preferably from 200 to 100000 parts by mass, more preferably from 400 to 75000 parts by mass, relative to 100 parts by mass of the binder from the viewpoint of productivity and operability.

The aqueous composition may optionally contain an active ingredient, an excipient, a disintegrant, and an additive. The powder composition may also optionally contain an additive.

Examples of the additive include a flavoring agent, a sweetener, a fluidizing agent, and a dissolution aid for the active ingredient.

Examples of the flavoring agent include menthol, peppermint oil, and vanillin.

Examples of the sweetener include aspartame, acesulfame potassium, sucralose, stevia, and thaumatin.

Examples of the fluidizing agent include light anhydrous silicic acid, hydrated silicon dioxide, and magnesium aluminometasilicate.

Examples of the dissolution aid of the active ingredient include organic acids such as fumaric acid, succinic acid, malic acid, tartaric acid and adipic acid; and alkali metal salts of the organic acids.

If necessary, two or more types of additives may be used. A commercial additive may be used. An amount of the additive is described below as the content in the tablet.

The granulation step may be carried out by using a granulator. Examples of the granulator include a fluidized bed granulator, a high shear granulator, a tumbling fluidized bed granulator, and a spray drying granulator.

When the granulation operation is explained by using a fluidized bed granulator as an example, a granulated product may be obtained by placing a powder composition containing at least one selected from group consisting of an active ingredient, an excipient and a disintegrant in the fluidized bed granulator, and granulating the powder composition, while adding (preferably spraying) an aqueous composition containing the predetermined binder and water.

In one of examples, a granulated product containing a sugar and/or sugar alcohol as an excipient and low-substituted hydroxypropyl cellulose having a hydroxypropoxy group content of 5 to 16% by mass as a disintegrant may be obtained in a granulation step of granulating a powder composition containing the sugar and/or sugar alcohol, while adding an aqueous composition containing the predetermined binder, water, the sugar and/or sugar alcohol and low-substituted hydroxypropyl cellulose having a hydroxypropoxy group content of 5 to 16% by mass to the powder composition. The sugar and/or sugar alcohol in the aqueous composition is present not only inside but also on the surface of the granulated product, and the presence on the surface may contribute to the modification of the surface. In another example, a granulated product may be obtained by a granulation step of granulating a powder composition containing a sugar and/or sugar alcohol and low substituted hydroxypropyl cellulose, while adding an aqueous composition containing a predetermined binder and water.

The average particle size of the granulated product varies depending on granulation conditions. It is preferably from 40 to 300 µm, more preferably from 45 to 250 µm, from the viewpoint of tabletability and reduced mass deviation between tablets. The average particle size of the granulated product may be determined by using a dry method based on the Fraunhofer diffraction theory with a laser diffraction type particle size distribution measuring apparatus (Master Sizer 3000 produced by Malvern) under the conditions of dispersion pressure of 2 bar and scattering intensity of 2 to 10%, where a value corresponding to a 50% cumulative size in the volume-based cumulative particle size distribution curve is used as the average particle size.

The obtained granulated product does not need to be further dried when a fluidized bed granulator capable of spraying and drying at the same time is used and drying is actually performed. However, it is preferable to perform drying in a known manner when drying is not actually performed or when a granulator incapable of drying is used.

The drying may be performed by using a dryer. Examples of the dryer include a fluidized bed dryer, a flash dryer, a box type dryer, a vibration dryer, a natural convection type constant temperature dryer, a forced convection type constant temperature dryer, and a forced convection type constant temperature and constant humidity dryer. The drying temperature is preferably from 40 to 80°C.

The water content of the dried granulated product is preferably 5.0% by mass or less, more preferably from 0.0 to 1.0% by mass from the viewpoint of tablet stability. The water content of the granulated product may be determined by using a heating and drying method moisture analyzer (MX-50 produced by A&D Company Ltd.) under the conditions of 5 g of granulated product, a heating temperature of 105°C, and a heating time of 60 minutes.

Next, the heat treatment and tableting step of subjecting the granulated product to heat treatment followed by tableting, or to tableting followed by heat treatment to obtain a tablet will be described.

If necessary, the granulated product may be converted to powder for tableting. The powder for tableting may be obtained by a mixing step of mixing the granulated product, which may be after or before the heat treatment, with at least one selected from the group consisting of an active ingredient, an excipient, a disintegrant, a binder, an additive and a lubricant described later. The addition of a binder to the granulated product is expected to enhance the moldability during tableting. When the granulated product after the heat treatment is used in the mixing step, the subsequent tableting may produce a tablet. When the granulated product before the heat treatment is used in the mixing step, the subsequent tableting followed by the heat treatment may produce a tablet. The mixing method is not particularly limited. The mixing may be done by using a mixer. Examples of the mixer include a V-shape rotating mixer, a ribbon mixer, a container mixer, and a tumbler mixer.

When there is nothing to be added to the granulated product, the granulated product itself may be used as powder for tableting.

The heat treatment may be carried out by using a dryer. Examples of the dryer include a fluidized bed dryer, a convection dryer, a box-type dryer, a vibration dryer, a natural convection type constant temperature dryer, a forced convection type constant temperature dryer, and a forced convection type constant temperature and constant humidity dryer. For example, when the forced convection type constant temperature dryer is used, the heat treatment may be carried out by placing a granulated product (including powder for tableting) or tablet in a container such as a vat, and leaving the container in the forced convection type constant temperature dryer having a predetermined internal temperature for a predetermined time.

The temperature in the heat treatment is preferably higher than 80°C, more preferably higher than 80°C and not higher than 180°C, still more preferably from 95 to 180°C, further still more preferably from 115 to 170°C, and particularly preferably 135 to 160°C, from the viewpoint of improvement of tablet hardness and disintegration time. The heat treatment time is preferably 5 minutes or more, more preferably from 5 to 120 minutes, and still more preferably from 10 to 90 minutes, from the viewpoint of improvement of tablet hardness and disintegration time.

When the granulated product is subjected to the heat treatment, the granulated product may be dried in a dryer and then subjected to the heat treatment in the same dryer without being taken out from the dryer, and alternatively, the granulated product may be subjected to drying and the heat treatment at the same time.

After the powder for tableting or a tablet is subjected to the heat treatment, it is preferably cooled to from 20 to 40°C. The temperature of the granulated product (including the powder for tableting) or tablet subjected to heat treatment may be measured by using a portable non-contact thermometer (PT-3LF produced by OPTEX FA Co., Ltd.).

The cooling method is not particularly limited. It may be left at room temperature or in a low temperature environment such as a refrigerator. The cooling may also be carried out in a desiccator or in a sealed container in order to avoid moisture absorption.

The heat treatment is preferably carried out after tableting from the viewpoint of improvement of the tablet hardness.

The tableting may be carried out by using a tableting machine. Examples of the tableting machine include a rotary tableting machine and a single-punch tableting machine. The tableting in the heat treatment and tableting step may be carried out in the presence or absence of a lubricant. It is preferably carried out in the presence of a lubricant from the viewpoint of preventing tableting failure.

As a method of adding a lubricant (hereinafter also referred to as "lubrication method"), an internal lubrication method or an external lubrication method may be selected.

According to the internal lubrication method, lubricant-containing powder is tableted with a tableting machine containing a pestle and a mortar wherein a lubricant is absent at powder-contact parts of both of the pestle and the mortar. On the other hand, according to the external lubrication method, lubricant-free powder or granulated product is tableted with a tableting machine containing a pestle and a mortar wherein a lubricant is present at a powder contact part of the pestle and/or a powder contact part of the mortar. The external lubrication method is preferable from the viewpoint of improvement of tablet hardness and disintegration time.

When a tablet is produced with a rotary tableting machine or a single-punch tableting machine in the internal lubrication method, lubricant-containing powder may be placed in a lubricant-free mortar and pressed by lubricant-free upper and lower pestles at a predetermined pressure.

When a tablet is produced with a rotary tableting machine connected to an external lubricant spray system (ELS-PI, Kikusui Seisakusho Ltd.) in the external lubrication method, lubricant-free powder or granulated product may be placed in a mortar and pressed by upper and lower pestles at a predetermined pressure, wherein a lubricants is present at each powder or granulated product contact part of the mortar and the pestle. Examples of a method of placing a lubricant at a powder contact part of a pestle and/or a powder contact part of mortar include spraying or coating.

Examples of the lubricant include talc; magnesium stearate; calcium stearate; sodium stearyl fumarate; sucrose fatty acid esters; waxes such as paraffin wax and carnauba wax; and hardened oils such as hardened castor oil, hardened rapeseed oil and hardened beef tallow oil.

An amount of the lubricant in the internal lubrication method is preferably from 0.2 to 5.0 parts by mass, more preferably from 0.4 to 3.0 parts by mass, relative to 100 parts by mass of the lubricant-free tablet (i.e. granulated product or powder for tableting) from the viewpoint of suppression of tableting failure, disintegration and improvement of tablet hardness and disintegration time.

An amount of the lubricant in the external lubrication method is preferably from 0.01 to 2.0 parts by mass, more preferably from 0.05 to 1.0 parts by mass, relative to 100 parts by mass of the lubricant-free tablet (i.e. granulated product or powder for tableting) from the viewpoint of suppression of tableting failure, disintegration, and improvement of tablet hardness and disintegration time.

The tableting pressure is preferably from 20 to 400 MPa from the viewpoint of tablet hardness and tableting failure.

The content of the active ingredient in the tablet is not particularly limited. It is preferably from 0.01 to 98.90% by mass from the viewpoint of the drug effect or efficacy.

The content of the excipient in the tablet is not particularly limited. It is preferably from 0.00 to 98.89% by mass, more preferably 0.00 to 95.00% by mass, from the viewpoint of controlling the tablet hardness and disintegration time.

The content of the disintegrant in the tablet is preferably from 1.0 to 40.0% by mass, more preferably from 2.0 to 30.0% by mass, and still more preferably from 2.0 to 20.0% by mass from the viewpoint of disintegration and storage stability.

The content of the binder in the tablet is preferably from 0.10 to 10.00% by mass, more preferably from 0.10 to 6.00% by mass, and still more preferably from 0.15 to 5.00% by mass from the viewpoint of the bindability, disintegration and improvement of tablet hardness and disintegration time.

The content of the additive in the tablet is not particularly limited. It is preferably from 0 to 10% by mass from the viewpoint of controlling the flavor of the tablet, controlling the fluidity of the powder for tableting or controlling the dissolution of the active ingredient.

An active ingredient, an excipient, a disintegrant, a binder and an additive may be included in any of the powder composition and the aqueous composition within each content range in the tablet as described above. Alternatively, they may optionally be added to and mixed with the obtained granulated product. The binder may be included not only in the aqueous composition but also in the powder composition before the granulation. It is because of the fact that the powder composition may be granulated, while adding an aqueous composition containing a binder and water to the powder composition, and the obtained granulated product may be tableted to obtain a tablet.

The tablet diameter is preferably from 6 to 12 mm from the viewpoint of handling and administration.

The mass of tablet is preferably from 70 to 700 mg per tablet.

The hardness of the tablet is preferably 50N or more, more preferably froom 80 to 250N, from the viewpoint of preventing cracking, chipping or the like during packing, transporting, or removal of the tablet from the PTP sheet. Tablet hardness may be measured by using a tablet hardness meter (TBH-125 produced by ERWEKA GmbH) and be obtained as the maximum breaking strength when load is applied to the tablet in the diameter direction at a rate of 1 mm/sec until the tablet breaks.

The disintegration time of the tablet is preferably within 5 minutes, more preferably within 3 minutes, and still more preferably within 1 minute, from the viewpoint of the onset of drug efficacy. The disintegration time of tablet may be determined by using a disintegration tester (NT-400 produced by Toyama Sangyo Co., Ltd.) in accordance with to the DisintegrationTest (test solution: water, no supplementary plate) of the Japanese Pharmacopoeia Seventeenth Edition.

### EXAMPLES

The invention will be explained with reference to Examples and Comparative Examples. It should not be construed that the invention is limited to or by Examples.

### <EXAMPLE 1>

An aqueous composition (hereinafter also referred to as "aqueous binder solution") was prepared by dissolving 6 g of polyvinyl alcohol (PVA) as a binder in 144 g of purified water. The polyvinyl alcohol was JF-05 produced by JAPAN VAM & POVAL CO., LTD. and had a degree of saponification of 98.5 mol % and a viscosity at 20°C of 5.0 mPa·s, as determined in a 4% by mass aqueous solution thereof.

Next, 60 g of acetaminophen (fine powder grade produced by Yamamoto Chemical Industry Co., Ltd.) as an active component and 219 g of D-mannitol (PEARLITOL 25C produced by Roquette Corporation) as an excipient were placed in a fluidized bed granulator (MP-01 produced by Powrex Corporation) and mixed at an air flow rate of 0.6 to 0.7 m³/min to obtain a powder composition.

In the granulator, the powder composition was granulated, while spraying the aqueous binder solution, under the conditions of a supply air temperature of 80°C, a discharge air temperature of from 35 to 38°C, an air flow rate of from 0.6 to 0.7 m³/min, a spray rate of 10 g/min, and a spray air pressure of 200 kPa to obtain a granulated product. The granulated product has an average particle size of 77 µm and the water content of 0.3% by mass.

Next, 228 g of the obtained granulated product was subjected to addition of 12 g of low-substituted hydroxypropyl cellulose (L-HPC) having a hydroxypropoxy group content of 11% by mass as a disintegrant and mixed to obtain powder for tableting.

Then, using a tabletop tableting machine (single-punch tableting machine HANDTAB-200 produced by Ichihashi Seiki Co., Ltd.) equipped with a pair of pestles having a diameter of 8 mm and a curvature radius of 12 mm and a mortar having a hole diameter of 8 mm, a small amount of magnesium stearate (vegetable grade produced by Taihei Chemical Industry Co., Ltd.) as a lubricant was applied to surfaces of the pestles and the mortar (the surfaces which the powder contacts) by using a cotton swab; 200 mg of the powder for tableting was filled; and the powder was tableted at a pressure of 5.0 kN (about 99.5 MPa) to obtain 200.2 mg of tableted product as a result of the external lubrication method.

Each tableted product was placed without overlapping each other in a petri dish, then subjected to heat treatment at 100°C for 60 minutes in a forced convection constant temperature and constant humidity oven (DKN402 produced by Yamato Scientific Co., Ltd.), and cooled by being left to stand in a desiccator of room temperature until the temperature of the tablet reached 25°C, thereby obtaining a tablet. The tablet hardness and disintegration time of the tablet was measured. Tablet composition, tablet hardness and disintegration time are shown in Tables 1 and 2. The ratio of with-heat-treatment to without-heat-treatment is a ratio of the measured value in Example 1 to the measured value in Comparative Example 1.

### <EXAMPLE 2>

A tablet was produced in the same manner as in Example 1 except that the temperature of the heat treatment of the tableted product was 120°C. The tablet hardness and disintegration time of the obtained tablet were measured in the same manner as in Example 1. The results are shown in Tables 1 and 2. The ratio of with-heat-treatment to without-heat-treatment is a ratio of the measured value in Example 2 to the measured value in Comparative Example 1.

### <EXAMPLE 3>

A tablet was produced in the same manner as in Example 1 except that the temperature of the heat treatment of the tableted product was 140°C. The tablet hardness and disintegration time of the obtained tablet were measured in the same manner as in Example 1. The results are shown in Tables 1 and 2. The ratio of with-heat-treatment to without-heat-treatment is a ratio of the measured value in Example 3 to the measured value in Comparative Example 1.

### <EXAMPLE 4>

A tablet was prepared in the same manner as in Example 3 except that a period of time of the heat treatment of the tableted product was 10 minutes. The tablet hardness and disintegration time of the obtained tablet were measured in the same manner as in Example 1. The results are shown in Tables 1 and 2. The ratio of with-heat-treatment to without-heat-treatment is a ratio of the measured value in Example 4 to the measured value in Comparative Example 1.

### <EXAMPLE 5>

A tablet was prepared in the same manner as in Example 3 except that a period of time of the heat treatment of the tableted product was 30 minutes. The tablet hardness and disintegration time of the obtained tablet were measured in the same manner as in Example 1. The results are shown in Tables 1 to 2. The ratio of with-heat-treatment to without-heat-treatment is a ratio of the measured value in Example 5 to the measured value in Comparative Example 1.

### <EXAMPLE 6>

The granulated product obtained in the same manner as in Example 1 was spread thinly on a stainless steel vat, then subjected to heat treatment at 140°C for 60 minutes in a forced convection constant temperature and constant humidity oven (DKN402 produced by Yamato Scientific Co., Ltd.), and cooled by being left to stand in a desiccator of room temperature until the temperature of the granulated product reached 25°C, thereby obtaining a heat-treated granulated product.

The 228 g of the heat-treated granulated product was subjected to addition of 12 g of low-substituted hydroxypropyl cellulose having a hydroxypropoxy group content of 11% by mass as a disintegrant, and mixed to obtain powder for tableting.

Then, the powder for tableting was tableted in the same manner as in Example 1 to obtain 200.2 mg of tableted product. The tableted product was used as a tablet without subjecting to the heat treatment. The tablet hardness and disintegration time of the obtained tablet were measured in the same manner as in Example 1. The results are shown in Tables 1 and 2. The ratio of with-heat-treatment to without-heat-treatment is a ratio of the measured value in Example 6 to the measured value in Comparative Example 1.

### <EXAMPLE 7>

The 228 g of the granulated product obtained in the same manner as in Example 1 was mixed with 12 g of low-substituted hydroxypropyl cellulose having a hydroxypropoxy group content of 11% by mass as a disintegrant, and then further mixed with 1.2 g of magnesium stearate (vegetable grade produced by Taihei Chemical Industry Co., Ltd.) as a lubricant, thereby obtaining powder for tableting.

Then, using a tableting machine (single-punch tableting machine HANDTAB-200 produced by Ichihashi Seiki Co., Ltd.) equipped with a pair of pestles having a diameter of 8 mm, a curvature radius of 12 mm and a mortar with a hole diameter of 8 mm, 201 mg of powder for tableting was filled and tableted at a pressure of 5.0 kN (about 99.5 MPa) to obtain 201 mg of tableted product as a result of the inner lubrication method.

Subsequently, the tableted product was subjected to heat treatment at 140°C for 60 minutes in a forced convection constant temperature and constant humidity oven (DKN402 produced by Yamato Scientific Co., Ltd.), and then cooled by being left to stand in a desiccator of room temperature until the temperature of the tableted product reac0hed 25°C, thereby obtaining a tablet. The tablet hardness and disintegration time of the obtained tablet were measured in the same manner as in Example 1. The results are shown in Tables 1 and 2. The ratio of with-heat-treatment to without-heat-treatment is a ratio of the measured value in Example 7 to the measured value in Comparative Example 2.

### <EXAMPLE 8>

The granulated product obtained in the same manner as in Example 1 was heat-treated at 140°C for 60 minutes in a forced convection constant temperature and constant humidity oven (DKN402 produced by Yamato Scientific Co., Ltd.), and then cooled by being left to stand in a desiccator of room temperature until the temperature of the granulated product reached 25°C, thereby obtaining a heat-treated granulated product.

The 228 g of the heat-treated granulated product was mixed with 12 g of low-substituted hydroxypropyl cellulose having a hydroxypropoxy group content of 11% by mass as a disintegrant, and then mixed with 1.2 g of magnesium stearate (vegetable grade produced by Taihei Chemical Industry Co., Ltd.) as a lubricant to obtain powder for tableting.

The powder for tableting was tableted in the same manner as in Example 7 to obtain 201 mg of tableted product. The 201 mg of tableted product without the heat treatment was used as a tablet as a result of the internal lubrication method. The tablet hardness and disintegration time of the obtained tablet were measured in the same manner as in Example 1. The results are shown in Tables 1 and 2. The ratio of with-heat-treatment to without-heat-treatment is a ratio of the measured value in Example 8 to the measured value in Comparative Example 2.

### <EXAMPLE 9>

A tablet was produced in the same manner as in Example 3 except that 3 g of polyvinyl alcohol having a degree of saponification of 98.5 mol% and a viscosity at 20°C of 5.0 mPa·s, as determined in a 4% by mass aqueous solution thereof, was dissolved in 147 g of purified water to obtain an aqueous binder solution, and 222 g of D-mannitol was placed in the fluidized bed granulator. The obtained granulated product had an average particle size of 57 µm, and a water content of 0.3% by mass. The tablet hardness and disintegration time of the obtained tablet were measured in the same manner as in Example 1. The results are shown in Tables 1 and 2. The ratio of with-heat-treatment to without-heat-treatment is a ratio of the measured value in Example 9 to the measured value in Comparative Example 3.

### <EXAMPLE 10>

A tablet was produced in the same manner as in Example 3 except that 9 g of polyvinyl alcohol having a degree of saponification of 98.5 mol % and a viscosity at 20°C of 5.0 mPa·s, as determined in a 4% by mass aqueous solution thereof, was dissolved in 141 g of purified water to obtain an aqueous binder solution, and 216 g of D-mannitol was placed in the fluidized bed granulator. The obtained granulated product had an average particle size of 106 µm, and a water content of 0.3% by mass. The tablet hardness and disintegration time of the obtained tablet were measured in the same manner as in Example 1. The results are shown in Tables 1 and 2. The ratio of with-heat-treatment to without-heat-treatment is a ratio of the measured value in Example 10 to the measured value in Comparative Example 4.

### <EXAMPLE 11>

A tablet was produced in the same manner as in Example 3 except that 6 g of polyvinyl alcohol (JP-05 produced by Japan VAM & POVAL Co., Ltd.) having a degree of saponification of 88.0 mol % and a viscosity at 20°C of 5.0 mPa·s, as determined in a 4% by mass aqueous solution thereof, was used as the aqueous binder solution. The obtained granulated product had an average particle size of 83 µm, and the water content was 0.3% by mass. The tablet hardness and disintegration time of the obtained tablet were measured in the same manner as in Example 1. The results are shown in Tables 1 and 2. The ratio of with-heat-treatment to without-heat-treatment is a ratio of the measured value in Example 11 to the measured value in Comparative Example 5.

### <EXAMPLE 12>

A tablet was produced in the same manner as in Example 3 except that the D-mannitol was not used and the mixing at an air flow rate of 0.6 to 0.7 m³/min was not carried out. The obtained granulated product had an average particle size of 87 µm, and a water content of 0.2% by mass. The tablet hardness and disintegration time of the obtained tablet were measured in the same manner as in Example 1. The results are shown in Tables 1 and 2. The ratio of with-heat-treatment to without-heat-treatment is a ratio of the measured value in Example 12 to the measured value in Comparative Example 6.

### <EXAMPLE 13>

A tablet was produced in the same manner as in Example 3 except that 60 g of theophylline (produced by SHIRATORI Pharmaceutical Co., Ltd.) was used as an active ingredient and 219 g of D-mannitol was placed. The obtained granulated product had an average particle size of 79 µm, and a water content of 0.2% by mass. The tablet hardness and disintegration time of the obtained tablet were measured in the same manner as in Example 1. The results are shown in Tables 1 and 2. The ratio of with-heat-treatment to without-heat-treatment is a ratio of the measured value in Example 13 to the measured value in Comparative Example 7.

### <EXAMPLE 14>

A tablet was produced in the same manner as in Example 3 except that 228 g of the obtained granulated product was subjected to mixing with 12 g of sodium starch glycolate (Primojel produced by the DFE Pharma) as a disintegrant. The tablet hardness and disintegration time of the obtained tablet were measured in the same manner as in Example 1. The results are shown in Tables 1 and 2. The ratio of with-heat-treatment to without-heat-treatment is a ratio of the measured value in Example 14 to the measured value in Comparative Example 8.

### <EXAMPLE 15>

A tablet was produced in the same manner as in Example 3 except that 228 g of the obtained granulated product was mixed with 12 g of croscarmellose sodium (Ac-Di-Sol produced by FMC Corporation). The tablet hardness and disintegration time of the obtained tablet were measured in the same manner as in Example 1. The results are shown in Tables 1 and 2. The ratio of with-heat-treatment to without-heat-treatment is a ratio of the measured value in Example 15 to the measured value in Comparative Example 9.

### <EXAMPLE 16>

A tablet was produced in the same manner as in Example 3 except that 6 g of hydroxypropyl methyl cellulose having a methoxy group content of 29.0% by mass and a hydroxypropoxy group content of 9.0% by mass, and a viscosity at 20°C of 6.0 mPa·s, as determined in a 2% by mass aqueous solution thereof, was used as a binder to form an aqueous binder solution. The obtained granulated product had an average particle size of 58 µm, and a water content of 0.3% by mass. The tablet hardness and disintegration time of the obtained table was measured by the same manner as in Example 1. The results are shown in Tables 1 and 2. The ratio of with-heat-treatment to without-heat-treatment is a ratio of the measured value in Example 16 to the measured value in Comparative Example 10.

### <EXAMPLE 17>

A tablet was produced in the same manner as in Example 3 except that 6 g of hydroxypropyl cellulose (HPC-SL produced by Nippon Soda Co., Ltd.) having a hydroxypropoxy group content of 64% by mass was used as a binder to form an aqueous binder solution. The obtained granulated product had an average particle size of 63 µm, and a water content of 0.2% by mass. The tablet hardness and disintegration time of the obtained tablet were measured in the same manner as in Example 1. The results are shown in Tables 1 and 2. The ratio of with-heat-treatment to without-heat-treatment is a ratio of the measured value in Example 17 to the measured value in Comparative Example 11.

### <EXAMPLE 18>

A tablet was produced in the same manner as in Example 3 except that 6 g of polyvinylpyrrolidone (K30 produced by Wako Pure Chemical Industries, Ltd.) having a K-value of 30 was used as a binder to form an aqueous binder solution. The obtained granulated product had an average particle size of 48 µm, and a water content of 0.2%. The tablet hardness and disintegration time of the obtained tablet were measured in the same manner as in Example 1. The results are shown in Tables 1 and 2. The ratio of with-heat-treatment to without-heat-treatment is a ratio of the measured value in Example 18 to the measured value in Comparative Example 12.

### <EXAMPLE 19>

The 0.8g of polyvinyl alcohol (JF-10 produced by Japan VAM & POVAL Co., Ltd.), as a binder, having a degree of saponification of 98.5 mol % and a viscosity at 20°C of 30.0 mPa·s, as determined in a 4% by mass aqueous solution thereof, and 60 g of D-mannitol (PEARLITOL 25C produced by Roquette Corporation) as an excipient were dissolved in 499.2 g of purified water, and then mixed with 40 g of low-substituted hydroxypropyl cellulose having a hydroxypropoxy group content of 8%as a disintegrant to obtain an aqueous composition which was an aqueous dispersion.

Then, 299.2 g of D-mannitol (PEARLITOL 25C produced by Roquette Corporation) as an excipient was placed in a fluidized bed granulator, and granulated, while spraying the aqueous dispersion thereto, under the conditions of a supply air temperature of 60°C, a discharge air temperature of 25 to 28°C, an air flow rate of 0.6 to 0.7 m³/min, a spray rate of 12 g/min, and a spray air pressure of 150 kPa to obtain a granulated product. The obtained granulated product had an average particle size of 61 µm, and a water content of 0.5% by mass.

Next, 192 g of the obtained granulated product was mixed with 48 g of acetaminophen (fine powder grade produced by Yamamoto Chemical Industry Co., Ltd.) as an active ingredient to obtain powder for tableting. The powder was tableted in the same manner as in Example 1 to produce 200.2 mg of tableted product to be heat-treated.

Subsequently, the tableted product was heat-treated at 140°C for 60 minutes in a forced convection constant temperature and constant humidity oven (DKN402 produced by Yamato Scientific Co., Ltd.), and then cooled by being left to stand in a desiccator until the temperature of the tableted product reached 25°C, thereby obtaining a tablet. The tablet hardness and disintegration time of the obtained tablet were measured in the same manner as in Example 1. The results are shown in Tables 1 and 2. The ratio of with-heat-treatment to without-heat-treatment is a ratio of the measured value in Example 19 to the measured value in Comparative Example 13.

### <Comparative Example 1>

A tablet was produced in the same manner as in Example 1 except that the tableted product was not heat-treated. The tablet hardness and disintegration time of the produced table were measured in the same manner as in Example 1. The results are shown in Tables 1 and 2.

### <Comparative Example 2>

A tablet was produced in the same manner as in Example 7 except that the tableted product was not heat-treated. The tablet hardness and disintegration time of the produced tablet were measured in the same manner as in Example 7. The results are shown in Tables 1 and 2.

### <Comparative Example 3>

A tablet was produced in the same manner as in Example 9 except that the tableted product was not heat-treated. The tablet hardness and disintegration time of the produced tablet were measured in the same manner as in Example 9. The results are shown in Tables 1 and 2.

### <Comparative Example 4>

A tablet was produced in the same manner as in Example 10 except that the tableted product was not heat-treated. The tablet hardness and disintegration time of the produced tablet were measured in the same manner as in Example 10. The results are shown in Tables 1 and 2.

### <Comparative Example 5>

A tablet was produced in the same manner as in Example 11 except that the tableted product was not heat-treated. The tablet hardness and disintegration time of the produced tablet were measured in the same manner as in Example 11. The results are shown in Tables 1 and 2.

### <Comparative Example 6>

A tablet was produced in the same manner as in Example 12 except that the tableted product was not heat treated. The tablet hardness and disintegration time of the produced tablet was measured in the same manner as in Example 12. The results are shown in Tables 1 and 2.

### <Comparative Example 7>

A tablet was produced in the same manner as in Example 13 except that the tableted product was not heat-treated. The tablet hardness and disintegration time of the produced table were measured in the same manner as in Example 13. The results are shown in Tables 1 and 2.

### <Comparative Example 8>

A tablet was produced in the same manner as in Example 14 except that the tableted product was not heat-treated. The tablet hardness and disintegration time of the produced tablet were measured in the same manner as in Example 14. The results are shown in Tables 1 and 2.

### <Comparative Example 9>

A tablet was produced in the same manner as in Example 15 except that the tableted product was not heat-treated. The tablet hardness and disintegration time of the produced tablet were measured in the same manner as in Example 15. The results are shown in Tables 1 and 2.

### <Comparative Example 10>

A tablet was produced in the same manner as in Example 16 except that the tableted product was not heat-treated. The tablet hardness and disintegration time of the produced tablet were measured in the same manner as in Example 16. The results are shown in Tables 1 and 2.

### <Comparative Example 11>

A tablet was produced in the same manner as in Example 17 except that the tableted product was not heat-treated. The tablet hardness and disintegration time of the produced tablet were measured in the same manner as in Example 17. The results are shown in Tables 1 and 2.

### <Comparative Example 12>

A tablet was produced in the same manner as in Example 18 except that the tableted product was not heat-treated. The tablet hardness and disintegration time of the produced tablet were measured in the same manner as in Example 18. The results are shown in Tables 1 and 2.

### Comparative Example 13

A tablet was produced in the same manner as in Example 19 except that the tableted product was not heat-treated. The tablet hardness and disintegration time of the produced table t were measured in the same manner as in Example 19. The results are shown in Tables 1 and 2.

**Table 1**

| | active ingredient | | excipient | disintegrant | | | binder | | | | |
|---|---|---|---|---|---|---|---|---|---|---|---|
| | acetaminophen | theophylline | mannitol | L-HPC | sodium starch glycolate | croscarmellose sodium | PVA degree of saponification of 98.5 mol% | PVA degree of saponification of 88.0 mol% | hydroxypropyl methyl cellulose | hydroxypropyl cellulose | polyvinylpyrrolidone |
| | (mass%) | (mass%) | (mass%) | (mass%) | (mass%) | (mass%) | (mass%) | (mass%) | (mass%) | (mass%) | (mass%) |
| Example1 | 20.00 | 0.00 | 73.00 | 5.0 | 0.0 | 0.0 | 2.00 | 0.00 | 0.00 | 0.00 | 0.00 |
| Example2 | 20.00 | 0.00 | 73.00 | 5.0 | 0.0 | 0.0 | 2.00 | 0.00 | 0.00 | 0.00 | 0.00 |
| Example3 | 20.00 | 0.00 | 73.00 | 5.0 | 0.0 | 0.0 | 2.00 | 0.00 | 0.00 | 0.00 | 0.00 |
| Example4 | 20.00 | 0.00 | 73.00 | 5.0 | 0.0 | 0.0 | 2.00 | 0.00 | 0.00 | 0.00 | 0.00 |
| Example5 | 20.00 | 0.00 | 73.00 | 5.0 | 0.0 | 0.0 | 2.00 | 0.00 | 0.00 | 0.00 | 0.00 |
| Example6 | 20.00 | 0.00 | 73.00 | 5.0 | 0.0 | 0.0 | 2.00 | 0.00 | 0.00 | 0.00 | 0.00 |
| Example7 | 20.00 | 0.00 | 73.00 | 5.0 | 0.0 | 0.0 | 2.00 | 0.00 | 0.00 | 0.00 | 0.00 |
| Example8 | 20.00 | 0.00 | 73.00 | 5.0 | 0.0 | 0.0 | 2.00 | 0.00 | 0.00 | 0.00 | 0.00 |
| Example9 | 20.00 | 0.00 | 74.00 | 5.0 | 0.0 | 0.0 | 1.00 | 0.00 | 0.00 | 0.00 | 0.00 |
| Example10 | 20.00 | 0.00 | 72.00 | 5.0 | 0.0 | 0.0 | 3.00 | 0.00 | 0.00 | 0.00 | 0.00 |
| Example11 | 20.00 | 0.00 | 73.00 | 5.0 | 0.0 | 0.0 | 0.00 | 2.00 | 0.00 | 0.00 | 0.00 |
| Example12 | 93.00 | 0.00 | 0.00 | 5.0 | 0.0 | 0.0 | 2.00 | 0.00 | 0.00 | 0.00 | 0.00 |
| Example13 | 0.00 | 20.00 | 73.00 | 5.0 | 0.0 | 0.0 | 2.00 | 0.00 | 0.00 | 0.00 | 0.00 |
| Example14 | 20.00 | 0.00 | 73.00 | 0.0 | 5.0 | 0.0 | 2.00 | 0.00 | 0.00 | 0.00 | 0.00 |
| Example15 | 20.00 | 0.00 | 73.00 | 0.0 | 0.0 | 5.0 | 2.00 | 0.00 | 0.00 | 0.00 | 0.00 |
| Example16 | 20.00 | 0.00 | 73.00 | 5.0 | 0.0 | 0.0 | 0.00 | 0.00 | 2.00 | 0.00 | 0.00 |
| Example17 | 20.00 | 0.00 | 73.00 | 5.0 | 0.0 | 0.0 | 0.00 | 0.00 | 0.00 | 2.00 | 0.00 |
| Example18 | 20.00 | 0.00 | 73.00 | 5.0 | 0.0 | 0.0 | 0.00 | 0.00 | 0.00 | 0.00 | 2.00 |
| Example19 | 20.00 | 0.00 | 71.84 | 8.0 | 0.0 | 0.0 | 0.16 | 0.00 | 0.00 | 0.00 | 0.00 |
| Comp.Ex.1 | 20.00 | 0.00 | 73.00 | 5.0 | 0.0 | 0.0 | 2.00 | 0.00 | 0.00 | 0.00 | 0.00 |
| Comp.Ex.2 | 20.00 | 0.00 | 75.00 | 5.0 | 0.0 | 0.0 | 2.00 | 0.00 | 0.00 | 0.00 | 0.00 |
| Comp.Ex.3 | 20.00 | 0.00 | 74.00 | 5.0 | 0.0 | 0.0 | 1.00 | 0.00 | 0.00 | 0.00 | 0.00 |
| Comp.Ex.4 | 20.00 | 0.00 | 72.00 | 5.0 | 0.0 | 0.0 | 3.00 | 0.00 | 0.00 | 0.00 | 0.00 |
| Comp.Ex.5 | 20.00 | 0.00 | 73.00 | 5.0 | 0.0 | 0.0 | 0.00 | 2.00 | 0.00 | 0.00 | 0.00 |
| Comp.Ex.6 | 93.00 | 0.00 | 0.00 | 5.0 | 0.0 | 0.0 | 2.00 | 0.00 | 0.00 | 0.00 | 0.00 |
| Comp.Ex.7 | 0.00 | 20.00 | 73.00 | 5.0 | 0.0 | 0.0 | 2.00 | 0.00 | 0.00 | 0.00 | 0.00 |
| Comp.Ex.8 | 20.00 | 0.00 | 73.00 | 0.0 | 5.0 | 0.0 | 2.00 | 0.00 | 0.00 | 0.00 | 0.00 |
| Comp.Ex.9 | 20.00 | 0.00 | 73.00 | 0.0 | 0.0 | 5.0 | 2.00 | 0.00 | 0.00 | 0.00 | 0.00 |
| Comp.Ex.10 | 20.00 | 0.00 | 73.00 | 5.0 | 0.0 | 0.0 | 0.00 | 0.00 | 2.00 | 0.00 | 0.00 |
| Comp.Ex.11 | 20.00 | 0.00 | 73.00 | 5.0 | 0.0 | 0.0 | 0.00 | 0.00 | 0.00 | 2.00 | 0.00 |
| Comp.Ex.12 | 20.00 | 0.00 | 73.00 | 5.0 | 0.0 | 0.0 | 0.00 | 0.00 | 0.00 | 0.00 | 2.00 |
| Comp.Ex.13 | 20.00 | 0.00 | 76.64 | 3.2 | 0.0 | 0.0 | 0.16 | 0.00 | 0.00 | 0.00 | 0.00 |

| | | | | | | | | | | | |
|---|---|---|---|---|---|---|---|---|---|---|---|
| * D-mannitol was used as "Mannitol". | | | | | | | | | | | |

**Table 2**

| | heat treatment | | | lubrication | amount of lubricant | tablet properties | | ratio of with-heat-treatment to without-heat-treatment | |
|---|---|---|---|---|---|---|---|---|---|
| | target | temperature | time | | | | | | |
| | | | | | | tablet hardness | disintegration time | tablet hardness | disintegration time |
| | | (°C) | (min) | - | (parts by mass) | (N) | (sec) | (%) | (%) |
| Example1 | tableted product | 100 | 60 | external | 0.1 | 115 | 97 | 134 | 84 |
| Example2 | tableted product | 120 | 60 | external | 0.1 | 122 | 68 | 142 | 59 |
| Example3 | tableted product | 140 | 60 | external | 0.1 | 141 | 42 | 164 | 36 |
| Example4 | tableted product | 140 | 10 | external | 0.1 | 119 | 41 | 138 | 35 |
| Example5 | tableted product | 140 | 30 | external | 0.1 | 138 | 41 | 160 | 35 |
| Example6 | granulated product | 140 | 60 | external | 0.1 | 88 | 31 | 102 | 27 |
| Example7 | tableted product | 140 | 60 | internal | 0.5 | 122 | 128 | 151 | 89 |
| Example8 | granulated product | 140 | 60 | internal | 0.5 | 87 | 35 | 107 | 24 |
| Example9 | tableted product | 140 | 60 | external | 0.1 | 97 | 20 | 152 | 37 |
| Example10 | tableted product | 140 | 60 | external | 0.1 | 158 | 187 | 174 | 37 |
| Example11 | tableted product | 140 | 60 | external | 0.1 | 167 | 43 | 186 | 36 |
| Exampe12 | tableted product | 140 | 60 | external | 0.1 | 99 | 29 | 157 | 71 |
| Exampe13 | tableted product | 140 | 60 | external | 0.1 | 131 | 30 | 138 | 15 |
| Example14 | tableted product | 140 | 60 | external | 0.1 | 132 | 101 | 165 | 80 |
| Example15 | tableted product | 140 | 60 | external | 0.1 | 129 | 12 | 161 | 29 |
| Example16 | tableted product | 140 | 60 | external | 0.1 | 118 | 32 | 159 | 94 |
| Example17 | tableted product | 140 | 60 | external | 0.1 | 188 | 26 | 251 | 90 |
| Example18 | tableted product | 140 | 60 | external | 0.1 | 201 | 33 | 226 | 100 |
| Example19 | tableted product | 140 | 60 | external | 0.1 | 82 | 13 | 152 | 81 |
| Comp.Ex.1 | no treatment | - | - | external | 0.1 | 86 | 116 | | |
| Comp.Ex.2 | no treatment | - | - | internal | 0.5 | 81 | 144 | - | - |
| Comp.Ex.3 | no treatment | - | - | external | 0.1 | 64 | 54 | - | - |
| Comp.Ex.4 | no treatment | - | - | external | 0.1 | 91 | 510 | - | - |
| Comp.Ex.5 | no treatment | - | - | external | 0.1 | 90 | 120 | - | - |
| Comp.Ex.6 | no treatment | - | - | external | 0.1 | 63 | 41 | - | - |
| Comp.Ex.7 | no treatment | - | - | external | 0.1 | 95 | 198 | - | - |
| Comp.Ex.8 | no treatment | - | - | external | 0.1 | 80 | 127 | - | - |
| Comp.Ex.9 | no treatment | - | - | external | 0.1 | 80 | 41 | - | - |
| Comp.Ex.10 | no treatment | - | - | external | 0.1 | 74 | 34 | - | - |
| Comp.Ex.11 | no treatment | - | - | external | 0.1 | 75 | 29 | - | - |
| Comp.Ex.12 | no treatment | - | - | external | 0.1 | 89 | 33 | - | - |
| Comp.Ex.13 | no treatment | - | - | external | 0.1 | 54 | 16 | - | - |

| | | | | | | | | | |
|---|---|---|---|---|---|---|---|---|---|
| * The amount of the lubricant is expressed as parts by mass relative to 100 parts by mass of the power for tableting containing no lubricant. | | | | | | | | | |

In Examples 1 to 5 where a tableted product produced in the presence of lubricant in the external lubrication method was heat-treated to obtain a tablet, the tablet hardness increased and the disintegration time decreased. Thus, both of the tablet hardness and the disintegration time were improved in Examples 1 to 5, compared with those in Comparative Example 1, where a tableted product produced in the presence of lubricant in the external lubrication method was not heat-treated and was used as a tablet. It is evident from the results of Examples 1 to 3 that the improvement of the tablet hardness and the disintegration time is greater as the temperature of the heat treatment is higher. It is evident from the results of Examples 3 to 5 that the improvement of the tablet hardness is greater as the period of time for the heat treatment is longer.

Further, in Example 7, where a tableted product produced by tableting lubricant-containing powder obtained in the internal lubrication method was heat-treated to obtain a tablet, both of the tablet hardness and the disintegration time were improved.

It is evident from the results of Examples 3 and 7 that when the tableted product is heat-treated, the external lubrication method provides higher improvement in both of tablet hardness and disintegration time than the internal lubrication method.

Further, in Example 6, where a tableted product produced by tableting the heat-treated granulated product in the presence of lubricant in the external lubrication method was not heat-treated and was used as a tablet, and in Example 8, where a tableted product produced by tableting after addition of a lubricant to the heat-treated granulated product in the internal lubrication method was not heat-treated and used as a tablet, the disintegration time could be improved, while maintaining the tablet hardness.

A tableted product is in the state where a predetermined binder and powders such as an active ingredient, an excipient and a disintegrant are compacted and contacted each other. When the tableted product is heat-treated, it is considered that the predetermined binder becomes softened to increase the contact area with the powders for stronger binding, thereby resulting in the increased tablet hardness (see Examples 1 to 5, 7, and 9 to 19). On the other hand, when the granulated product is heated, it is considered that a strong binding cannot not be formed because there are many voids between the predetermined binder and the powders, thereby resulting in no increase of the tablet hardness (see Examples 6 and 8).

In general, when a tablet absorbs water, a predetermined binder dissolves to develop viscosity, thereby deteriorating water conduction and delaying disintegration time. In Examples 1 to 15 and 19, where polyvinyl alcohol was used as a binder, it is considered that the development of viscosity was suppressed and the disintegration time was shortened because of increased crystallinity and lowered solubility in water after softening the polyvinyl alcohol in the heat treatment of tableted or granulated product. On the other hand, in Examples 16, 17, and 18, where a binder other than polyvinyl alcohol was used, it is considered that the solubility in water and the disintegration time were maintained because of no change in the crystallinity after softening the predetermined binder in the heat treatment of tableted product.

When a tableted product produced by tableting after addition of a lubricant by the internal lubrication method is heat-treated, it is considered that the lubricant is contained inside the tableted product and spreads widely inside the tableted product during the heat treatment, so that the hydrophobicity of the tableted product increases, thereby inhibiting improvement of the disintegration time (Example 7). On the other hand, when a tableted product produced by tableting in the external lubrication method is heat-treated, it is considered that the above-described inhibiting effect does not occur because of lack of the lubricant inside of the tableted product (Example 3).

When tableting is carried out after the heat treatment of the granulated product, the lubricant added in either the internal lubrication method or the external lubrication method is not heat-treated, so that the above-mentioned inhibiting effect is not generated (Examples 6 and 8).

It is evident from the results of Examples 9 to 11 that both of tablet hardness and disintegration time are improved regardless of the amount of binder added and the degree of saponification of polyvinyl alcohol. In Example 12 in the absence of an excipient, Example 13 with the use of a different type of active ingredient, and Examples 14 and 15 with the use of a different type of disintegrant, the tablet hardness and disintegration time were improved. It is considered that none of the excipient, the active ingredient and the disintegrant brings the improvement, but the heat treatment of granulated product or tableted product, each containing a predetermined binder, brings the improvement.

In Example 16 with the use of hydroxypropyl methyl cellulose, Example 17 with the use of hydroxypropyl cellulose, and Example 18 with the use of polyvinylpyrrolidone, each as the predetermined binder, the tablet hardness increases, while maintaining the disintegration time. The polyvinyl alcohol is excellent in improvements of both of the tablet hardness and the disintegration time.

It is evident from the results of Example 19 that even addition of active ingredient to the composite granulated product containing an excipient, a predetermined binder and a disintegrant improves the tablet hardness and disintegration time.

## Claims

1. A method for producing a tablet, comprising:
a granulation step of granulating a powder composition comprising at least one selected from the group consisting of an active ingredient, an excipient and a disintegrant, while adding an aqueous composition comprising a binder and water to the powder composition, to obtain a granulated product; and
a heat treatment and tableting step of subjecting the granulated product to heat treatment and then tableting, or to tableting and then heat treatment, to obtain a tablet; wherein the binder is selected from the group consisting of polyvinyl alcohol, hydroxypropyl cellulose, polyvinylpyrrolidone, hydroxypropyl methyl cellulose, methyl cellulose, sodium carboxymethylcellulose, polyvinyl alcohol/acrylic acid/methyl methacrylate copolymers, and vinylpyrrolidone/vinyl acetate copolymers.

2. The method for producing a tablet according to claim 1, wherein the polyvinyl alcohol has a degree of saponification of 78.0 mol% or more.

3. The method for producing a tablet according to claim 1 or 2, wherein the excipient is selected from the group consisting of sugars, sugar alcohols, starches, dextrins, powdered cellulose, microcrystalline cellulose, calcium carbonate, calcium phosphate, and calcium sulfate.

4. The method for producing a tablet according to any one of claims 1 to 3,
wherein the disintegrant is selected from the group consisting of low-substituted hydroxypropyl cellulose, starches, partly pregelatinized starch, sodium starch glycolate, carmellose, carmellose calcium, croscarmellose sodium, microcrystalline cellulose, and crospovidone.

5. The method for producing a tablet according to any one of claims 1 to 4,
wherein the tableting in the heat treatment and tableting step is carried out in an external lubricating method in which a lubricant is applied to a pestle surface and/or a mortar surface of a tableting machine.

6. The method for producing a tablet according to any one of claims 1 to 5,
wherein the excipient is sugar and/or sugar alcohol, and the disintegrant is low-substituted hydroxypropyl cellulose having a hydroxypropoxy group content of 5 to 16% by mass.

7. The method for producing a tablet according to any one of claims 1 to 6, wherein a temperature in the heat treatment is higher than 80°C.
